# EUROPEAN PATENT SPECIFICATION

(11) **EP 1 925 299 B1**
(45) Date of publication and mention of the grant of the patent: **03.12.2014**
(21) Application number: 06782486.2
(22) Date of filing: 08.08.2006
(51) Int. Cl.: A61K 9/70, A61K 31/405, A61K 47/02, A61K 47/32, A61P 29/00

(54) **ADHESIVE PATCH LESS IRRITATIVE TO SKIN**
KLEBEPFLASTER MIT GERINGERER HAUTREIZUNG
TIMBRE ADHESIF MOINS IRRITANT POUR LA PEAU

(30) Priority: 10.08.2005 JP 2005231593
(43) Date of publication of application: 28.05.2008
(73) Proprietor: Hisamitsu Pharmaceutical Co., Inc., Tosu-shi, Saga 841-0017 (JP)
(72) Inventor: SAEKI, Masakazu, Tosu-shi, Saga 8410017 (JP); WAKAMATSU, Masato, Tosu-shi, Saga 8410017 (JP); YOSHINAGA, Takaaki, Tosu-shi, Saga 8410017 (JP)
(74) Representative: Raynor, Stuart Andrew
(86) International application number: PCT/JP2006/315660
(87) International publication number: WO 2007/018210

(56) References cited:
- EP-A1- 1 238 664
- EP-A2- 0 307 187
- WO-A1-01/68061
- JP-A- 05 271 056
- JP-A- 06 000 201
- JP-A- 06 125 977
- JP-A- 06 145 052
- JP-A- 06 154 304
- JP-A- 07 316 045
- JP-A- 07 330 602
- JP-A- 10 130 145
- JP-A- 10 158 186
- JP-A- 2002 000 639
- JP-A- 2003 095 958
- JP-A- 2004 256 396
- JP-A- 2005 075 755
- US-A- 5 429 591
- US-A1- 2003 124 174
- US-A1- 2003 149 383
- DATABASE WPI Week 199425 Thomson Scientific, London, GB; AN 1994-206336 XP002665895, & JP 6 145052 A (HISAMITSU PHARM CO LTD) 24 May 1994 (1994-05-24)
- DATABASE WPI Week 199828 Thomson Scientific, London, GB; AN 1998-316676 XP002665937, & JP 10 114646 A (TOKO YAKUHIN KOGYO KK) 6 May 1998 (1998-05-06)
- DATABASE WPI Week 200224 Thomson Scientific, London, GB; AN 2002-182022 XP002665938, & JP 2001 302502 A (SAITAMA DAIICHI SEIYAKU KK) 31 October 2001 (2001-10-31)

## Description

### Technical Field

The present invention relates to an adhesive patch less irritative to the skin, particularly, the present invention relates to an adhesive patch containing an active ingredient which causes less skin irritation. More particularly, the present invention relates to an adhesive patch comprising an adhesive base agent and supporting body, which is characterized by containing 3% by mass to 30 % by mass of low-molecular-weight polyisobutylene, aluminum hydroxide, a thermoplastic rubber and edetic acid or a salt thereof, but not substantially containing water.

### Background of the Invention

To date, adhesive patches for external use are for example plaster patches, cataplasms, tape patches and the like, which are preparations wherein adhesive polymers containing a drug are directly or indirectly spread over a supporting body such as a flexible fabric, nonwoven fabric or a plastic film and the like.

Such adhesive patches for external use cause adverse effects such as generation of stress to the skin during its application, physical action such as pulling the skin and/or hair when removing the patch, chemical action caused by irritating components of raw materials, accumulation of sweat and moisture resulting from the prolonged application, and reddening, rash and the like caused by physiological effect such as sweating, those were the problems to be solved. Major reason for these problems is considered to be destruction of tissues on the surface of the skin caused by stress to the skin during the application of an existing patch and pulling the skin and/or hair when removing the patch.

On the other hand, various experiments have been made to solve these problems. For example, a method of opening vent holes in a plaster is disclosed in Patent Literature 1. Also, containing chemicals for preventing rash or reducing skin irritation is disclosed in Patent Literature 2, Patent Literature 3, Patent Literature 4, etc. A method for reducing skin irritation by particular treatment to the base agent is disclosed in Patent Literature 5. Further, adding water-absorbing polymers in order to eliminate physiological factors of rash by sudor, sweating and the like, and physical factors of rash by such as pulling the hair on removing is disclosed in Patent Literature 6. However, in any of these methods, an adhesive patch decreased the adhesion by containing additive components and resulted in unsuitable for practical use, the production process was complicated, and the effect was insufficient. As described above, various methods have been tested; however, they were not for practical use. Consequently, an ideal patch for external use which is not causing rash has yet to be appeared.

On the other hand, in transdermal application, skin irritation such as pruritus, flush, rash, pain, eczema, skin inflammation and the like caused by a composition of an adhesive patch or drug itself is rarely recognized, and remedies for those have been desired. Particularly, various researches have been made for application of a patch containing indomethacin, an antiinflammatory analgesic drug, to the skin (see Patent Literature 7 to 9).

Incidentally, titanium oxide has been used as a drug additive, such as a coating agent or an ultraviolet absorber; for example, an adhesive patch containing titanium oxide in the supporting body as an inorganic ultraviolet shielding agent has been reported (see Patent Literature 10). Also, it is reported that titanium oxide has effect in such as treating and improving various external injuries, cutaneous diseases and symptoms and the like or effective in improving the skin; therefore, a medical product comprising titanium oxide which is contained in various base agents has been used (see Patent Literature 11). Further, an adhesive patch comprising a styrene-isoprene-styrene block copolymer, an alicyclic saturated hydrocarbon resin, isopropyl myristate, liquid paraffin, titanium oxide, diiron trioxide, dibutylhydroxytoluene, 1-menthol, and indomethacin intermittently laminated with a layer of adhesive agent has been reported as an adhesive patch which reduces generation of dermatopathy, having excellent adhesion and providing high cutaneous absorption (see Patent Literature 12).

Furthermore, it has been reported that an obstruction improved adhesive patch can be obtained by containing a metal oxide such as titanium oxide in a matrix for an adhesive patch (see Patent Literature 13), and it has also been reported that an adhesive patch with creaseless film-like supporting body can be obtained by containing a poorly-water-soluble cross-linking agent such as titanium oxide or dry aluminum hydroxide gel in aqueous adhesive layer of the adhesive patch (see Patent Literature 14).

Patent Literature 15 relates to a patch with less skin stimulation, excellent long term storage stability, heat stability and having favourable tack during use. Such a patch comprises a styrene-isoprene-styrene block copolymer, polyisobutylene, tackifier, plasticizer and pharmaceutically effective ingredient, in which two or more kinds of polyisobutylene of different average molecular weight are used in combination and the viscosity of the adhesive of the patch is between 1500 and 30,000 poise (at 60°C) and the tack of the patch is from 5 to 200 g/10 mm.

Patent Literature 16 relates to an anti-inflammatory-containing plaster comprising 5-40% by mass of a styrene-isoprene-styrene block copolymer, 1-25% by mass of a high molecular weight polysiobutylene, 0.5-24% by mass of a low molecular weight polyisobutylene, 3-50% by mass of a tackifier, 20-70% by mass of a plasticizer, 0.01-7% by mass of a dispersant, and 0.1-8% by mass of an anti-inflammatory having a carboxyl group or a salt thereof, but which contains no L-menthol, wherein the medicament release rate at one hour after the start of test is 20-64% by mass and the medicament release rate at three hours after the start of test is 40-93% by mass according to the water-releasing test using a rotating cylinder described in the release test as prescribed in United State Pharmacopoeia.
Patent Literature 1: Utility model JP-B-58-52251
Patent Literature 2: JP-A-58-4721
Patent Literature 3: JP-A-60-56911
Patent Literature 4: JP-A-60-23312
Patent Literature 5: JP-B-59-19528
Patent Literature 6: JP No. 2632838
Patent Literature 7: JP-A-10-114646
Patent Literature 8: JP-A-10-130145
Patent Literature 9: JP-A-2001-302502
Patent Literature 10: WO 01/068061
Patent Literature 11: JP-A-2003-95958
Patent Literature 12: JP-A-8-133973
Patent Literature 13: JP-A-10-287587
Patent Literature 14: JP-A-2003-95929
Patent Literature 15: EP 1 238 664 A1
Patent Literature 16: US 2003/0149383 A1

### Disclosure of the Invention

### Problem to be solved by the Invention

In view of the above circumstances, the present invention has been achieved to provide an adhesive patch for external use wherein rash caused thereby is reduced and a drug itself contained therein has less skin irritation.

### Means of solving the Problems

The present inventors have made earnest studies to achieve said objective and found that an adhesive base agent containing 3 % by mass to 30 % by mass of low-molecular-weight polyisobutylene, aluminum hydroxide, a thermoplastic rubber and edetic acid or a salt thereof but not substantially containing water reduces rash, and significantly reduces skin irritation caused by a drug when the drug is contained in the adhesive base agent, and the invention was completed.

That is, the present invention relates to an adhesive patch comprising an adhesive base agent and supporting body, characterized by containing 3 % by mass to 30 % by mass of a low-molecular-weight polyisobutylene, aluminum hydroxide, a thermoplastic rubber und edetic acid or a salt thereof in the adhesive base agent, particularly, the adhesive patch is characterized by not substantially containing water.

Further, the present invention relates to an adhesive patch characterized by further comprising a drug in the adhesive base agent which is characterized by containing indomethacin as said drug.

Further, the present invention relates to an adhesive patch characterized in that the content ratio of said thermoplastic rubber, which is a styrene-isoprene-styrene block copolymer, and the low-molecular-weight polyisobutylene is from 60:40 to 85:15, preferably from 60:40 to 75:25.

More specifically, the present invention relates to the following:
(1) An adhesive patch comprising an adhesive base agent and a supporting body, characterized in that the adhesive base agent contains 3% by mass to 30% by mass of a low-molecular-weight polyisobutylene of molecular weight from 10,000 to 60,000, aluminium hydroxide, a thermoplastic rubber and edetic acid or a salt thereof, but does not substantially contain water,
   wherein the thermoplastic rubber is a styrene-isoprene-styrene block copolymer.
(2) The adhesive patch according to (1) as above, wherein the content of the low-molecular-weight polyisobutylene is from 6 % by mass to 30 % by mass.
(3) The adhesive patch according to (1) or (2) as above, wherein the content of the low-molecular-weight polyisobutylene is 11 % by mass to 30 % by mass.
(4) The adhesive patch according to any of (1) to (3) as above, wherein the content ratio of the thermoplastic rubber and the low-molecular-weight polyisobutylene is 60:40 to 75:25.
(5) The adhesive patch according to any of (1) to (4) as above, wherein the content of the aluminum hydroxide in the adhesive base agent of the adhesive patch is 0.01 % by mass to 10 % by mass.
(6) The adhesive patch according to any of (1) to (5) as above, characterized by further containing a drug in the adhesive base agent of the adhesive patch.
(7) The adhesive patch according to (6) as above, wherein the drug is selected from the group consisting of a nonsteroidal antiinflammatory analgesic drug, a disease-modifying antirheumatic drug, a cytokine blocking drug, a smoking cessation adjuvant, and an antianginal drug.
(8) The adhesive patch according to (6) or (7) as above, wherein the drug is at least one selected from indomethacin, ketoprofen, diclofenac sodium, flurbiprofen, felbinac, ibuprofen, suprofen, tiaprofen, loxoprofen, celecoxib, lofecoxib, meloxicam, valdecoxib, nicotine, nitroglycerin and isosorbide dinitrate.
(9) The adhesive patch according to any of (6) to (8), wherein the drug is indomethacin.

An adhesive patch of the present invention is an adhesive patch wherein an adhesive base agent is directly or indirectly spread at one surface of a supporting body.

The adhesive base agent for the adhesive patch of the present invention comprises thermoplastic rubber which is a styrene-isoprene-styrene block copolymer and does not substantially contain water. Examples for further preferable thermoplastic rubber can include, but as an adhesive component not especially limited to, a high-molecular-weight substances such as polymers of silicone, styrene-butadiene copolymers, acrylic polymers, vinyl ether polymers, natural rubbers, polyisoprene polymers, polyurethanes, polyisobutylenes, acrylic acid polymers and the like. Preferable amount of content of these polymer substances contained in an adhesive base agent is 10 % by mass to 90 % by mass, particularly in the case of what is called diene-based thermoplastic rubber such as a natural rubber, a polyisoprene type, a styrene-isoprene-styrene type, a polyisobutylene type and the like, preferably be 10 % by mass to 50 % by mass.

It should be noted that an adhesive base agent in an adhesive patch of the present invention does not substantially contain water. The term "substantially" used herein means that there is not a step to add water intentionally to an adhesive base agent in the production processes, but not meant to exclude moisture contained in raw materials and the like or moisture absorbed by the adhesive base agent such as sweat and the like during the application.

An adhesive base agent of an adhesive patch of the present invention preferably contains low-molecular-weight polyisobutylene in a specified ratio to the total amount of the adhesive base agent. Low-molecular-weight polyisobutylene contained in a specific amount can control keratin layer to be exfoliated when removing the adhesive patch after the application, accordingly, physical irritation by exfoliation can be significantly reduced.

Preferable amount of low-molecular-weight polyisobutylene in an adhesive base agent is 3 % by mass to 30 % by mass, more preferably 6 % by mass to 20 % by mass. Also, content ratio of an adhesive component, preferably thermoplastic rubber, is preferably 40:60 to 85:15, more preferably 60: 40 to 75:25. When the content amount is over 30 % by mass, plaster reduces cohesive strength and the plaster residue remains on the skin, while the content amount below 3 % by mass increases skin irritation by physical effect produced when the patch is removed.

The low-molecular-weight polyisobutylene used herein means molecular weight of 10000 to 60000, and such polyisobutylene exhibits property of very viscous and adhesive semi-solid at normal temperature.

Such adhesive base agents can express significant effect of the adhesive patch of the present invention by containing aluminum hydroxide and/or titanium oxide therein. That is, the present invention is characterized by an adhesive base agent which does not contain water but contains preferably thermoplastic rubber as an adhesive component, comprising low-molecular-weight polyisobutylene of molecular weight 10000 to 60000, preferably a molecular weight 10000 to 40000, in an amount of 3 % by mass to 30 % by mass, preferably 6 % by mass to 30 % by mass, 11 % by mass to 30 % by mass, and more preferably 6 % by mass to 20 % by mass, 11 % by mass to 20 % by mass relative to total mass of the adhesive base agent, further comprising at least one component selected from the group consisting of aluminum hydroxide and titanium oxide.

The adhesive base agent of the present invention contains aluminum hydroxide. The adhesive patch containing such aluminum hydroxide can significantly reduce skin rash when applying the patch to the skin or removing the patch from the skin. Particularly, when an adhesive patch is containing a drug, skin irritation caused by the drug can also significantly be reduced.

The content amount of aluminum hydroxide and, optionally, titanium oxide in an adhesive base agent is preferably 0.01 % by mass to 10 % by mass for each, more preferably 0.1 % by mass to 8 % by mass, and even more preferably 0.5 % by mass to 5 % by mass. The content amount 0.01 % by mass or less tends to reduce effect of reducing skin rash which is undesirable. To the contrary, the content amount 10 % by mass or more reduces cohesion of the plaster and decreases adhesion, which is also undesirable.

Aluminum hydroxide and titanium oxide may be contained concurrently; however, combination of both is preferable for a drug particularly indomethacin, an antiinflammatory analgesic drug.

In a preferable embodiment of the present invention, an effect of an adhesive patch of the present invention can significantly be produced to reduce causing rash or skin irritation by using an adhesive base agent containing styrene-isoprene-styrene block copolymer as thermoplastic rubber and low-molecular-weight polyisobutylene in the above content amount but not substantially containing water, and further adding aluminum hydroxide and edetic acid or a salt thereof to the base agent.

In the present invention, adhesive base agent further comprises edetate. The content amount of edetate can be, but not limited to, a range from 0.01 % by mass to 5 % by mass, preferably from 0.1 % by mass to 5 % by mass and more preferably from 0.1 % by mass to 3 % by mass relative to total amount of the adhesive base agent. The edetate used herein can include, but not limited to, various edetate salts such as an alkali metal salt or an alkaline earth metal salt and the like, preferably an alkali metal salt particularly a sodium salt. An example of preferable edetate is disodium edetate.

If necessary, an adhesive base agent of the present invention can contain conventionally used tackifier, for example, a rosin type resin, [Ester gum(ARAKAWA CHEMICAL)], Hariester(HARIMA CHEMICAL), Pentalyn (Eastman Chemical), Foral (Eastman Chemical), a terpene type resin, [YS-Resin (YASUHARA CHEMICAL)], Picolyte (Loos & Dilworth), a petroleum resin, [Arkon (ARAKAWA CHEMICAL INDUSTRIES, LTD.), Rigaret (Eastman Chemical), Picolastic (Eastman Chemical), Escorez (Exxon), Wingtack (GOODYEAR), Quintone (ZEON), a phenolic resin, a xylene type resin and the like in an amount equal to or less than 50 % by mass.

However, when for example an acrylic type base agent is used as a polymer substance of an adhesive component, some has sufficient adhesiveness by themselves depending on the properties; therefore, the above tackifier is not necessary in such a case.

An adhesive base agent of an adhesive patch of the present invention can contain a drug. Examples of such a drug can include, but not limited to, an anti-inflammatory analgesic drug such as acetaminophen, phenacetin, mefenamic acid, diclofenac sodium, flufenamic acid, aspirin, sodium salicylate, methyl salicylate, glycol salicylate, aminopyrine, alclofenac, ibuprofen, naproxen, flurbiprofen, ketoprofen, amfenac sodium, mepirizole, indomethacin, piroxicam, loxoprofen, tiaprofen, acemetacin, ferbinac, sulindac, etodolac, tolmetin, ampiroxicam, azapropazone, valdecoxib, lofecoxib and the like; a steroidal antiinflammatory drug such as hydrocortisone, triamcinolone, dexamethasone, prednisolone and the like; a vasodilator drug such as diltiazem hydrochloride, pentaerythritol tetranitrate, isosorbide dinitrate, tradipil, nicorandil, nitroglycerin, prenylamine lactate, molsidomine, amyl nitrate, tolazoline hydrochloride, nifedipine and the like; a drug for arrhythmia such as procainamide hydrochloride, lidocaine hydrochloride, propranolol hydrochloride, alprenolol hydrochloride, atenolol, nadolol, metoprolol tartrate, ajmaline, disopyramide, mexiletine hydrochloride and the like; an antihypertensive agent such as ecarazine hydrochloride, indapamide, clonidine hydrochloride, bunitrolol hydrochloride, labetalol hydrochloride, captopril, guanabenz acetate, mebutamate, betanidine sulfate and the like; a cough medication and expectorant such as carbetapentane citrate, cloperastine, oxeladin tannate, clobutinol hydrochloride, clofedanol hydrochloride, noscapine hydrochloride, ephedrine hydrochloride, isoproterenol hydrochloride, clorprenaline hydrochloride, methoxyphenamine hydrochloride, procaterol hydrochloride, tulobuterol hydrochloride, clenbuterol hydrochloride, ketotifen fumarate and the like; an antineoplastic drug such as cyclophosphamide, fluorouracil, tegafur, mitomycin C, procarbazine hydrochloride, doxifluridine, ranimusutine and the like; a topical anesthetic such as ethyl aminobenzoate, tetracaine hydrochloride, procaine hydrochloride, dibucaine hydrochloride, oxybuprocaine hydrochloride, propitocaine hydrochloride and the like; a hormone drug such as propylthiouracil, thiamazole, metelonon acetate, estradiol, estriol, progesterone and the like; an antihistamine drug such as diphenhydramine hydrochloride, chlorpheniramine maleate, promethazine, cyproheptadine hydrochloride, diphenylpyraline hydrochloride and the like; an anticoagulant drug such as warfarin potassium, ticlopidine hydrochloride and the like; an antispasmodic drug such as atropine methylbromide, scopolamine and the like; a general anesthetic such as thiopental sodium, pentobarbital sodium and the like; a hypnotic-analgesic drug such as bromovalerylurea, amobarbital, phenobarbital and the like; an antiepileptic drug such as phenytoin sodium and the like; a stimulant. analeptic drug such as methamphetamine hydrochloride and the like; an antidinic such as difenidol hydrochloride, betahistine mesylate and the like; a drug for psychoneurosis such as chlorpromazine hydrochloride, thioridazine, meprobamate, imipramine hydrochloride, chlordiazepoxide, diazepam and the like; a skeletal muscle relaxant such as suxamethonium hydrochloride, eperisone hydrochloride and the like; an autonomic drug such as neostigmine bromide, bethanechol chloride and the like; an antiparkinsonism drug such as amantadine hydrochloride and the like; a diuretic drug such as hydroflumethiazide, isosorbide, furosemide and the like; a vasoconstrictor such as phenylephrine hydrochloride and the like; a respiratory stimulant such as lobeline bromide, dimorpholamine, naloxone hydrochloride and the like; a peptic antiulcer drug such as glycopyrronium bromide, proglumide, cetraxate hydrochloride, cimetidine, spizofurone and the like; a cholagogue such as ursodesoxycholic acid, osalmid and the like; a drug for urogenital organ and anus such as hexamine, sparteine, dinoprost, ritodrine hydrochloride and the like; a drug for parasitic skin diseases such as salicylic acid, cyclopiroxolamine, cloconazole hydrochloride and the like; a skin softener such as urea and the like; a vitamin drop such as calcitriol, thiamine hydrochloride, riboflavin sodium phosphate, pyridoxine hydrochloride, nicotinic acid amide, panthenol, ascorbic acid and the like; an inorganic formulation such as calcium chloride, potassium iodide, sodium iodide and the like; a hemostat such as etamsylate and the like; a drug for liver disease such as thiopronine and the like; a drug for habitual toxicosis such as cyanamide and the like; an arthrifuge such as colchicine, probenecid, sulfinpyrazone and the like; an antidiabetic such as tolbutamide, chlorpropamide, glymidine sodium, glypsol, buformine hydrochloride, insulin and the like; an antibiotic such as benzylpenicillin potassium, propicillin potassium, cloxacillin sodium, sodium ampicillin, bacampicillin hydrochloride, carbenicillin sodium, cephaloridine, sodium cefoxitin, erythromycin, chloramphenicol, tetracycline, kanamycin sulfate, cycloserine and the like; a chemotherapeutic drug such as isoniazid, pyrazinamide, ethionamide and the like; a narcotic such as morphine hydrochloride, codeine phosphate, cocaine hydrochloride, pethidine hydrochloride, fentanyl citrate and the like; a disease-modifying antirheumaic drug such as leflunomide, auranofin and the like; a cytokine blocking drug such as infliximab, etanercept, anakinra and the like; a smoking cessation adjuvant such as nicotine and the like; an antianginal drug such as nitroglycerin and the like.

A drug preferable to be contained in an adhesive base agent of an adhesive patch of the present invention is a drug which increases skin irritation by itself because an adhesive patch of the present invention can exhibit a significant effect. Examples of such drugs include a nonsteroidal type antiinflammatory analgesic drug such as indomethacin, ketoprofen, flurbiprofen, felbinac, diclofenac, ibuprofen, suprofen and the like. An adhesive patch of the present invention particularly an adhesive patch containing indomethacin exhibits a significant effect.

In addition to the above mentioned components, an adhesive patch of the present invention can also contain fillers, antioxidants, cross-linking agents, stabilizers, antiseptics, absorption promoters and the like accordingly.

Examples of a preferable antioxidant include tocopherol and ester derivatives thereof, ascorbic acid, ascorbyl stearate, nordihydroguaiaretic acid, dibutylhydroxytoluene, butylhydroxyanisol and the like.

Examples of a preferable filler include calcium carbonate, magnesium carbonate, silicate (for example, alminium silicate, magnesium silicate and the like), silicic acid, barium sulfate, calcium sulfate, calcium zincate, zinc oxide, titanium oxide and the like.

Examples of a preferable cross linking agent include organic cross linking agents such as a thermosetting resin (an amino resin, a phenol resin, an epoxy resin, an alkyd resin, unsaturated polyester and the like), an isocyanate compound, a block isocyanate compound, and inorganic cross linking agents such as metal or a metal compound and the like.

Examples for a preferable antiseptic include ethyl p-hydroxybenzoate, propyl p-hydroxybenzoate, butyl p-hydroxybenzoate and the like, and examples of a preferable ultraviolet absorbing agent include p-aminobenzoic acid derivatives, anthranilic acid derivatives, salicylic acid derivatives, coumarin derivatives, amine-acid compounds, imidazoline derivatives, pyridine derivatives, dioxane derivatives and the like.

Examples of a preferable absorption promoter include terpene oil such as d-limonene; and aliphatic acid esters such as glycerin monolaurate, glycerin monooleate, diethyl sebacate; aliphatic acids such as azone, pirotiodecane, oleic acid, lauric acid, myristic acid or derivatives thereof and the like.

Supporting body used in an adhesive patch of the present invention is selected from an elastic or non-elastic supporting body such as a film or sheet, for example, polyethylene, polypropylene, polybutadiene, an ethylene-vinyl acetate copolymer, polyvinylchloride, polyester (for example, polyethylene terephthalate(PET)), nylon, polyurethane and the like, or a porous body and a foamed body thereof, and paper, cloth, nonwoven fabric, woven fabric (including knitted fabric) and the like. Particularly, when the adhesive patch is applied to a highly mobile region of the body such as the joint, rash or skin irritation can be reduced by using woven fabric or nonwoven fabric which is elastic as a supporting body.

An adhesive patch of the present invention can be easily produced by a conventionally known method. For example, to produce a tape patch which has rubber-type adhesive agent as a base, firstly mix a base and other components such as softener and tackifier on heating at 120-160 °C by using a mixing machine such as a kneader, mixer and the like, subsequently adjust the mixture for formation of adhesive layer by adding and mixing medicinal properties at a temperature not thermally decompose the medicinal properties. The mixture can then be directly spread over a film as a supporting body to form an adhesive layer, or the mixture can be spread over a release coated paper or film and place a supporting body thereon to transfer the adhesive layer on the supporting body by pressure. To produce an acrylic tape patch which has acrylic adhesive base agent, dissolve or disperse a base, medicinal property and absorption promoter in a solvent; and apply the resulting coating solution to the surface of the supporting body; and dry to form adhesive base agent. A solvent used herein preferably be able to dissolve all the components such as base, medicinal property and the like; for example, aromatic hydrocarbons such as toluene, benzene, and xylene and the like; esters such as ethyl acetate and the like; and halogenated hydrocarbons such as carbon tetrachloride, chloroform, and methylene chloride and the like.

As aluminum hydroxide, without limiting type of crystal such as the number of crystal water or presence or absence thereof, anhydrous aluminum hydroxide, plate-like crystal aluminum hydroxide, aluminum hydroxide gel and the like can be used.

As titanium oxide, without limiting type of crystal, a rutile type or an anatase type and the like can be used.

### Advantageous effect of the Invention

An adhesive patch of the present invention can provide an adhesive patch for external use which can significantly reduce incidence of rash and has reduced skin irritation by a drug by containing low-molecular-weight polyisobutylene and at least one component selected from the group consisting of aluminum hydroxide and titanium oxide, in an adhesive base agent.

### Brief Description of the Drawings

Fig. 1 is a graph showing test results of containing aluminum hydroxide in an adhesive patch of the present invention.
Fig. 2 is a graph showing test results of containing titanium oxide in an adhesive patch of the present invention.
Fig. 3 is a graph showing test results of containing low-molecular-weight polyisobutylene in an adhesive patch of the present invention.

The present invention is further illustrated by the following examples but not limited to these examples.

### Example 1

### (Preparation of an adhesive patch)

Each components (% by mass) indicated as Examples 1-8 shown in Table 1 were homogenously mixed, and the mixture was laminated over a supporting body to cover an adhesive base agent which was spread over a polyester film by coater to the thickness of 100 µm then cut to a given shape.

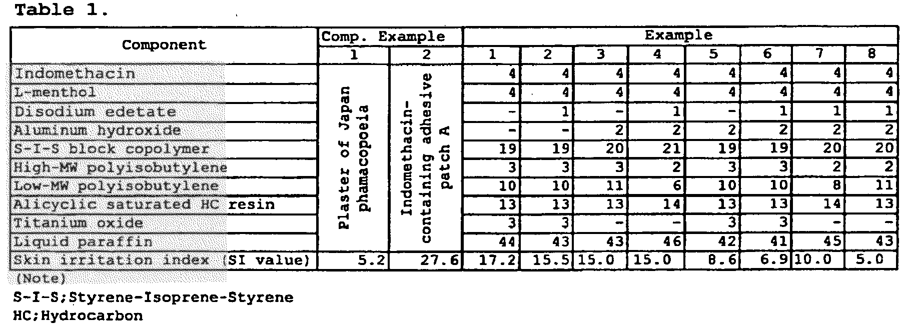

According to the above method (examples 1-8), an adhesive patch was produced, and skin irritancy of the adhesive patch was assessed by 48-hour patch test on 30 healthy male adult subjects.

A plaster of Japan Pharmacopoeia was used as Comparative Example 1, and a conventional indomethacin-containing adhesive patch (a content of indomethacin of 3.75 % by mass) was used as Comparative Examples 2 respectively.

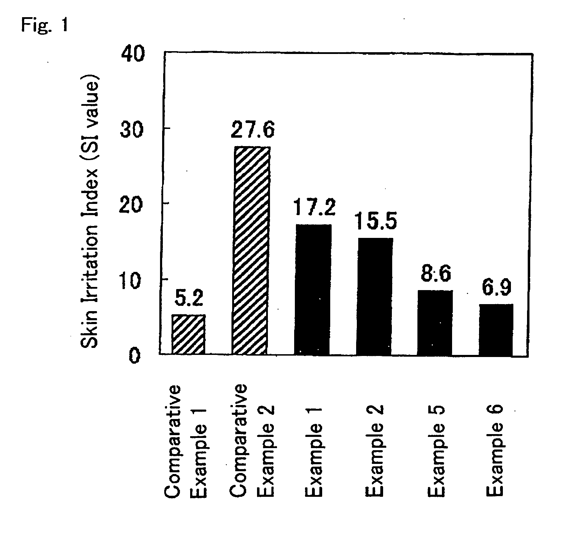

Each adhesive patch (size: circular form, 1.5cm in diameter) was applied to a subject on the back for 48 hours, and after removing the patch skin irritation index (SI value) was obtained. The skin irritation index was obtained by the Sugai equation (see "Skin Research" vol. 27, No. 4, Aug. 1985). The results are shown in Table 1, Fig. 1, Fig. 2 and Fig. 3. The vertical axis of each of the drawings shows the skin irritation index (SI value).

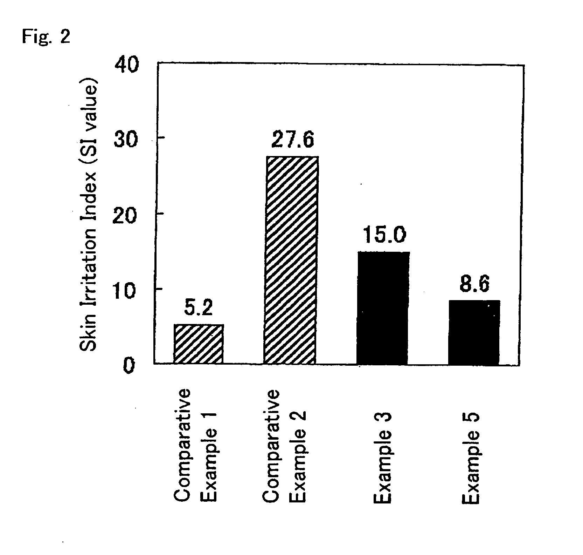

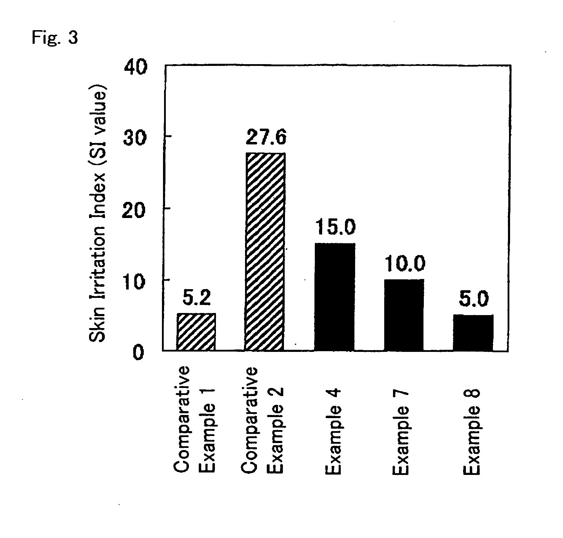

Fig. 1 shows test results on Examples 1 and 2 which did not contain aluminum hydroxide and Examples 5 and 6 which contained aluminum hydroxide. From these results, it was found that the skin irritation index (SI value) was reduced to half or less by containing aluminum hydroxide. It was also found that an adhesive patch containing only titanium oxide (Example 1 and 2) could reduce skin irritation index (SI value) to about 15-17 compared to comparative example 2 of 27.6 which contained indomethacin. Fig. 2 shows test results on Example 3 which did not contain titanium oxide and Example 5 which contained titanium oxide. From these results, it was found that the skin irritation index (SI value) was reduced from 15.0 to 8.6 by containing titanium oxide. It was also found that the skin irritation index (SI value) was reduced from 27.6 to 15.0 by containing aluminum hydroxide. As above, it was found that an adhesive patch of Example reduced skin irritation and incidence of rash by containing aluminum hydroxide and/or titanium oxide in an adhesive base agent. Also, as shown in Fig. 3, an adhesive patch of Example similarly reduced skin irritation index (SI value) by increasing content of low-molecular-weight polyisobutylene in an adhesive base agent. Further, it was found that the skin irritation index (SI value) was reduced by containing an edetate (see Example 3 and Example 8). Therefore, according to the present invention, an adhesive patch with sufficiently reduced skin rash can be provided.

### Industrial Applicability

The present invention provides an adhesive patch for external use which causes reduced incidence of rash and reduced skin irritation by a drug itself, and the present invention has industrial applicability in pharmaceutical industry or industry relating to health promotion.

## Claims

1. An adhesive patch comprising an adhesive base agent and a supporting body, **characterized in that** the adhesive base agent contains 3% by mass to 30% by mass of a low-molecular-weight polyisobutylene of molecular weight from 10,000 to 60,000, aluminium hydroxide, a thermoplastic rubber and edetic acid or a salt thereof, but does not substantially contain water,
wherein the thermoplastic rubber is a styrene-isoprene-styrene block copolymer.

2. The adhesive patch according to claim 1, wherein the content of the low-molecular-weight polyisobutylene is from 6% by mass to 30% by mass.

3. The adhesive patch according to claim 1 or 2, wherein the content of the low-molecular-weight polyisobutylene is 11 % by mass to 30% by mass.

4. The adhesive patch according to any of claims 1 to 3, wherein the content ratio of the thermoplastic rubber and the low-molecular-weight polyisobutylene is 60:40 to 75:25.

5. The adhesive patch according to any of claims 1 to 4, wherein the content of the aluminium hydroxide in the adhesive base agent of the adhesive patch is 0.01% by mass to 10% by mass.

6. The adhesive patch according to any of claims 1 to 5, **characterized by** further containing a drug in the adhesive base agent of the adhesive patch.

7. The adhesive patch according to claim 6, wherein the drug is selected from the group consisting of a nonsteroidal antiinflammatory analgesic drug, a disease-modifying antirheumatic drug, a cytokine blocking drug, a smoking cessation adjuvant, and an antianginal drug.

8. The adhesive patch according to claim 6 or 7, wherein the drug is at least one selected from indomethacin, ketoprofen, diclofenac sodium, flurbiprofen, felbinac, ibuprofen, suprofen, tiaprofen, loxoprofen, celecoxib, lofecoxib, meloxicam, valdecoxib, nicotine, nitroglycerin and isosorbide dinitrate.

9. The adhesive patch according to any of claims 6 to 8, wherein the drug is indomethacin.

## Patentansprüche

1. Klebepflaster, welches ein Haftgrundmittel und einen Tragkörper umfasst, **dadurch gekennzeichnet, dass** das Haftgrundmittel 3 Masseprozent bis 30 Masseprozent eines Polyisobutylens geringen Molekulargewichts mit einem Molekulargewicht von 10.000 bis 60.000, Aluminiumhydroxid, einen thermoplastischen Gummi und Äthylendiamintetraessigsäure oder ein Salz derselben enthält, aber im Wesentlichen kein Wasser enthält,
wobei der thermoplastische Gummi ein Styrol-Isopren-Styrol-Blockcopolymer ist.

2. Klebepflaster nach Anspruch 1, wobei der Anteil des Polyisobutylens geringen Molekulargewichts bei 6 Masseprozent bis 30 Masseprozent liegt.

3. Klebepflaster nach Anspruch 1 oder 2, wobei der Anteil des Polyisobutylens geringen Molekulargewichts bei 11 Masseprozent bis 30 Masseprozent liegt.

4. Klebepflaster nach einem der Ansprüche 1 bis 3, wobei das Anteilsverhältnis des thermoplastischen Gummis und des Polyisobutylens geringen Molekulargewichts bei 60:40 bis 75:25 liegt.

5. Klebepflaster nach einem der Ansprüche 1 bis 4, wobei der Anteil des Aluminiumhydroxids in dem Haftgrundmittel des Klebepflasters bei 0,01 Masseprozent bis 10 Masseprozent liegt.

6. Klebepflaster nach einem der Ansprüche 1 bis 5, **gekennzeichnet durch** weiteres Enthalten eines Arzneimittels in dem Haftgrundmittel des Klebepflasters.

7. Klebepflaster nach Anspruch 6, wobei das Arzneimittel aus der Gruppe bestehend aus einem nicht-steroidalen entzündungshemmenden Analgetikum, einem krankheitsmodifizierenden Antirheumatikum, einem zytokinblockierenden Arzneimittel, einem Raucherentwöhnungsadjuvans und einem antiangiösen Arzneimittel gewählt ist.

8. Klebepflaster nach Anspruch 6 oder 7, wobei das Arzneimittel mindestens eines gewählt aus Indomethacin, Ketoprofen, Diclofenac-Natrium, Flurbiprofen, Felbinac, Ibuprofen, Suprofen, Tiaprofen, Loxoprofen, Celecoxib, Lofecoxib, Meloxicam, Valdecoxib, Nikotin, Nitroglycerin und Isosorbiddinitrat ist.

9. Klebepflaster nach einem der Ansprüche 6 bis 8, wobei das Arzneimittel Indomethacin ist.

## Revendications

1. Timbre adhésif comprenant un agent de base adhésif et un corps de support, **caractérisé en ce que** l'agent de base adhésif contient 3 % en masse à 30 % en masse d'un polyisobutylène à faible masse moléculaire de masse moléculaire de 10 000 à 60 000, de l'hydroxyde d'aluminium, un caoutchouc thermoplastique et de l'acide édétique ou un sel de celui-ci, mais ne contient sensiblement pas d'eau,
dans lequel le caoutchouc thermoplastique est un copolymère séquencé de styrène-isoprène-styrène.

2. Timbre adhésif selon la revendication 1, dans lequel la teneur en polyisobutylène à faible masse moléculaire est de 6 % en masse à 30 % en masse.

3. Timbre adhésif selon la revendication 1 ou 2, dans lequel la teneur en polyisobutylène à faible masse moléculaire est de 11 % en masse à 30 % en masse.

4. Timbre adhésif selon l'une quelconque des revendications 1 à 3, dans lequel la proportion en teneur du caoutchouc thermoplastique et du polyisobutylène à faible masse moléculaire est de 60:40 à 75:25.

5. Timbre adhésif selon l'une quelconque des revendications 1 à 4, dans lequel la teneur en hydroxyde d'aluminium dans l'agent de base adhésif du timbre adhésif est de 0,01 % en masse à 10 % en masse.

6. Timbre adhésif selon l'une quelconque des revendications 1 à 5, **caractérisé en ce qu'**il contient en outre un médicament dans l'agent de base adhésif du timbre adhésif.

7. Timbre adhésif selon la revendication 6, dans lequel le médicament est choisi parmi le groupe constitué d'un médicament analgésique anti-inflammatoire non stéroïdien, d'un médicament antirhumatismal modificateur de la maladie, d'un médicament bloquant les cytokines, d'un adjuvant de désaccoutumance du tabac et d'un médicament antiangineux.

8. Timbre adhésif selon la revendication 6 ou 7, dans lequel le médicament est au moins l'un choisi parmi l'indométhacine, le kétoprofène, le diclofénac de sodium, le flurbiprofène, le felbinac, l'ibuprofène, le suprofène, le tiaprofène, le loxoprofène, le célécoxib, le lofécoxib, le méloxicam, le valdécoxib, la nicotine, la nitroglycérine et l'isosorbide dinitrate.

9. Timbre adhésif selon l'une quelconque des revendications 6 à 8, dans lequel le médicament est l'indométhacine.
